# EUROPEAN PATENT APPLICATION

(11) **EP 3 210 979 A1**
(43) Date of publication of application: **30.08.2017**
(21) Application number: 16157731.7
(22) Date of filing: 26.02.2016
(51) Int. Cl.: C07D 307/52, C07D 307/89, C07C 249/12, C07C 249/16, C07C 251/48, C07C 251/86, C07C 45/51, C07C 47/544, C07C 51/27, C07C 63/26

(54) **AROMATIC COMPOUNDS FROM FURANICS**

(71) Applicant: Nederlandse Organisatie voor toegepast- natuurwetenschappelijk onderzoek TNO, 2595 DA 's-Gravenhage (NL)
(72) Inventor: CROCKATT, Marc, 2595 DA 's-Gravenhage (NL); URBANES, Jan Harm, 2595 DA 's-Gravenhage (NL)
(74) Representative: V.O.

(57) **Abstract**

Described are methods for preparing benzene carboxylic acids, esters and anhydrides from furanic compounds by reaction with a dienophile, wherein the furanic compounds are reacted with a hydrazine and/or oxime and then reacted with a dienophile.

## Description

The present invention relates to the production of benzene carboxylic acids, especially from renewable biomass.

Benzene dicarboxylic acids and their derivatives, e.g. ortho-phthalic acid (also referred to herein as phthalic acid), meta-phthalic acid (also referred to herein as isophthalic acid) and para-phthalic acid (also referred to herein as terephthalic acid) and their esters, are used on large scale for the production of plasticizers, synthetic fibers, (plastic) bottles, in particular PET bottles, fire-retardant materials, (polyester) resins and the like. Currently, commercialized processes for the preparation of benzene dicarboxylic acids typically involve the oxidation of hydrocarbons that are based on fossil fuels such as naphtalene or ortho-xylene (for phthalic acid), *m*-xylene (for isophthalic acid) and p-xylene (for terephthalic acid).

It is desirable that the production processes of benzene carboxylic acids, such as benzene dicarboxylic acids, that are currently based on using chemicals from fossil fuels are replaced or complemented with bio-based production processes, i.e. processes for which the required chemicals originate from a biomass feedstock. Typically, biomass that is suitable for production of chemicals comprises one or more of the following components: oils, fats, lignin, carbohydrate polymers (e.g. starch, cellulose, hemicellulose, pectin, inulin), sucrose, sugar alcohols (e.g. erythritol).

These components can be converted into building blocks for further processing. For instance, carbohydrates can be converted into furanic compounds that may serve as a starting point for the production of phthalic acids.

A current research aim is providing a process for the large scale production of benzene carboxylic acids from renewable feedstock, in particular from lignocellulose-based materials. A first step may be breaking down biomass into lignin, cellulose and hemicellulose, followed by e.g. hydrolysis of the cellulose and dehydration of the obtained sugar to provide furanic compounds. The catalytic dehydration of in particular C₅ sugars generally yields furfural and catalytic dehydration of C₆ sugars may yield 5-hydroxymethylfurfural (5-HMF). The presence of an electron-withdrawing aldehyde group deactivates these furanic compounds for Diels-Alder (DA) reactions. Accordingly, furfural and 5-HMF are for example subjected to decarbonylation and/or hydrogenation and/or hydrogenolysis before the DA reaction, also because furfural and 5-HMF are unstable. After a Diels-Alder reaction of 2-methylfurane (2-MF) to give an aromatic compound, oxidation of the methyl group would require harsh conditions.

A reaction of 2-MF and maleic anhydride (MA) is shown in Scheme 1 in Angew. Chem. Int. Ed. 2016, 55, 1368-1371. An intermediate hydrogenation step of the oxabicyclic adduct is used, wherein H₂ is consumed to give a hydrogenated DA adduct that is aromatized in a tandem catalytic reaction. However, the latter reaction is aselective and yields a complex mixture of products. The formation of mono-acids is atom inefficient. Further oxidation of the still present methyl group into a carboxyl group would generally require harsh conditions, as mentioned. It would also involve introducing oxygen atoms from an external source such as an oxidizing salt, thereby incurring additional costs.

Another approach is decarbonylation of furfural to furan (as mentioned in for example WO 2014/064070), followed by a Diels Alder reaction with MA (as described for example in US 2014/0142279) to give the oxabicyclic adduct, followed by ring opening to phthalic anhydride. This reaction is not atom efficient; in particular in the decarbonlylation step a carbon atom is lost. It has limited yield because the oxabicyclic adduct is susceptible to retro-reaction. The oxabicyclic adduct can also be hydrogenated and aromatized. As described in Angew. Chem. Int. Ed. 2016, 55, 1368-1371, the reaction yields a complex mixture of products of which about 57% is aromatic. Moreover, this reaction involves the same disadvantage that the process comprises hydrogenation and dehydrogenation.

A reaction of furfural dimethylhydrazone with maleic anhydride is described in WO 2007/136640, wherein the formed isobenzofuran is further reacted with 3-aminopiperidine-2,6-dione hydrochloride. The reaction is also mentioned in *PNAS* 112(12) E1471-1479 for the preparation of thalidomide analogues, wherein it is followed by a reaction with rac-3-amino-2,3-dioxopiperidine. The reaction is also mentioned in J. Org. Chem. 49(21) 4099-4101 (1984) and J. Org. Chem. 53(6) 1199-1202 (1988). None of these documents is directed to biomass-derived compounds and the conversion of an aldehyde group of a furanic compound into a carboxylic group or ester group, to provide benzene carboxylic acids and/or esters. These are generally bulk chemicals.

Particularly desirable is the production of terephthalic acid from renewable biomass. A process for the production thereof from 2,5-dimethylfuran is given in WO 2014/043468.

An object of the invention is to provide a method for the production of benzene carboxylic acid compounds and esters from biomass-derived furanic compounds that, for example, is more atom efficient, involves fewer process steps, and may have higher selectivity and yield, and allows for use of relatively inexpensive catalysts.

It was surprisingly found that one or more of these advantages can be achieved at least in part by converting the aldehyde group of a furan carbaldehyde into a hydrazone or oxime group and reacting the hydrazone or oxime compound with a dienophile to give an aromatic compound, suitable followed by conversion of the hydrazone or oxime group in to a carboxylic or ester group by e.g. hydrolysis and oxidation.

In an aspect, the present invention relates to:
A method of preparing a compound having a backbone structure according to formula (I), 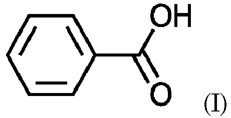
or esters or anhydrides thereof from biomass-derived compounds, comprising reacting a biomass-derived compound having a backbone structure according to formula (II): 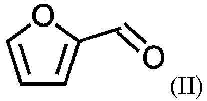 with a compound with formula (III): 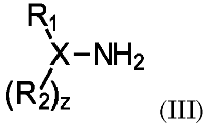
wherein X is O and z is 0, or X is N and z is 1,
wherein R₁ and R₂ are each independently an optionally substituted and/or
heteroatom containing hydrocarbyl group or a link to a heterogeneous support, and
R₂ can also be hydrogen,
to give a compound with a backbone structure according to formula (IV): 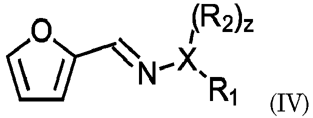
and reacting the compound with a backbone structure according to formula (IV) with a dienophile to give a compound with a backbone structure according to formula (V): 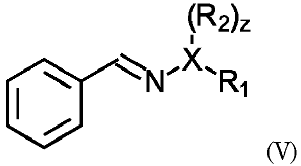 and converting said compound into the compound of formula (I) by hydrolysis and oxidation.

Hence, the method involves, in an aspect, preparing a benzene carboxylic compound, or an ester thereof, from a furan carbaldehyde, by condensation of the aldehyde group on the furanic ring with a hydrazine compound or a hydroxylamine, to provide the corresponding hydrazone or oxime group on the furanic ring, and reacting the hydrazone or oxime furanic compound with a dienophile, to form an aromatic hydrazone or oxime, suitably through ring opening of a bicyclic adduct as intermediate, and conversion of the hydrazone or oxime to give the corresponding carboxylic acid or ester, through hydrolysis and oxidation. For example, the hydrazone or oxime is hydrolysed to the corresponding benzaldehyde, followed by oxidation of the aldehyde group into carboxylic acid. In this way the carbonyl carbon atom of the furan carbaldehyde is maintained in the obtained benzene carboxylic acid.

Advantageously, by converting the aldehyde group into a hydrazone or oxime group, the oxidation state of the carbon atom of the aldehyde group is not altered while elegantly the hydrazone or oxime group is electron-donating, thereby rendering the furan more reactive for DA reactions. In particular, the second heteroatom (X in formula III) suitably has a lone pair of electrons that has p-orbital overlap with the furan ring, for instance through resonance interaction. Providing the hydrazone or oxime group may result in an increase of the HOMO (highest occupied molecular orbital) energy of the furan ring, relative to that of e.g. furfural. In this way, the reaction with the dienophile and ring opening may be enabled or promoted, and the aldehyde functionality can be restored in the aromatic product without oxidation, using for example hydrolysis. A further oxidation of the aldehyde to carboxylic acid can be relatively mild, and likewise oxidation of a hemiacetal to ester. This can be contrasted by the harsh conditions necessary for oxidation of a methyl group into carboxylic acid, if for instance a methyl furan compound was subjected to Diels-Alder reaction.

The compound that is reacted with the compound with formula (III) is a furanic compound with formula (II), for example a furan carbaldehyde, that is for instance biomass derived.

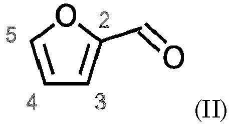

The furan with formula (II) is optionally substituted at any one or more of the 3, 4 and 5 position, in particular at the 5 position, for example only at the 5 position, for instance with one or more groups independently selected from the group consisting of optionally substituted or heteroatom-containing hydrocarbonyl, for example a C₁ - C₂₀ hydrocarbonyl, preferably C₁ - C₈, such as linear or branched alkyl, alkenyl, alkynyl, cycloalkyl, aryl, aralkyl and alkaryl, more in particular methyl; and F, Cl, Br, I, -CH₂F, -CH₂Cl, -CH₂Br, -CH₂I, -CN, -NO₂, -CHO, -CO₂H and esters thereof, -CH₂NH₂ and secondary, tertiary and quaternary amines or amides thereof, and -CH₂OH and esters or ethers thereof; wherein said heteroatoms are optionally selected from O, N, S, and P, and wherein said substituents are optionally from the mentioned substituents.

In an embodiment, the compound with formula (II) is optionally substituted with one or more groups independently selected from the group consisting of -CH₂OR, -(C=O)R, -(C=O)OR, wherein R is hydrogen or hydrocarbonyl, preferably C₁ - C₆ hydrocarbonyl. Optionally, the compound is only substituted with such groups, for example only at the 5 position, or is obtained from such compound. Optionally, a mixture of compounds with formula (II) is used.

Hence, the method uses an optionally substituted furan-2-carbaldehyde. For example, the method uses furfural, furan-2,5-dicarbaldehyde, 5-hydroxy-methylfurfural, methoxymethylfurfural and chloromethylfurfural, or a mixture thereof since these compounds can be obtained from C₅ and C₆ sugars under acidic conditions. In such case, the method may comprise an optional step of providing these compounds with one or more substituents, and subsequent reaction with the compound with formula (III).

Preferably, the compound with formula (II) is biomass-derived, more preferably derived from C₅ and C₆ sugars obtained from renewable biomass. Optionally, the method comprises preparing a compound with formula (II) from sugars and/or from biomass. The biomass may for instance comprise one or more of the following components: oils, fats, lignin, carbohydrate polymers (e.g. starch, cellulose, hemicellulose, pectin, inulin), sucrose, sugar alcohols (e.g. erythritol).

| The method may comprise converting the biomass into furanics for example in a one or a two-step procedure. In a two-step procedure biomass is typically first pre-treated to hydrolyse the cellulose and hemicellulose in order to obtain free sugars, i.e. non-polymerized sugars. This hydrolysis can be conducted by means of enzymatic fermentation, acid catalysis, and the like. Said sugars are then converted in a separate step into one or more furanics by means of acid-catalysed dehydrations. The method may also optionally comprise dehydration of sugars to yield furan derivatives such as furfural and 5-hydroxymethylfurfural (5-HMF), for example as described in WO 2007/146636, or converting cellulose and hemicellulose directly into one or more furanics by acid-catalysis. Given the limited variety of sugar units in biomass, only a limited variety of furanics are obtained directly from biomass. Therefore, in the present invention the variety of furanics is also limited in some embodiments. Typically, 5-HMF is obtained from cellulose and furfural is obtained from hemicellulose.

The compound with formula (II) may also be obtained from other sources than biomass, such as from petrochemical processes or for example from carbohydrate-containing waste streams.

In the method, the compound with formula (II) is reacted with a compound with formula (III): 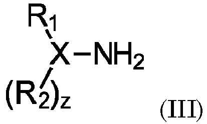
wherein X is O and z is 0 or X is N and z is 1,
wherein R₁ and R2 are each independently an optionally substituted and/or optionally heteroatom containing hydrocarbyl group, or a link to a heterogeneous support and R₂ can also be hydrogen.

Preferably, R₁ is such that the heteroatom X is attached to a sp³ hybridized carbon, and R₂, if present, is preferably either hydrogen or is attached to the heteroatom X by a sp³ hybridized carbon. In this way, X preferably has only single bonds with R₁ and, if present, R₂. Lack of conjugation of X with R₁ and R₂ may contribute to the activation of the furanic ring for Diels-Alder reactions.

R₁ and R₂ are for example independently an unsubstituted or substituted C₁ - C₂₀ or C₁ - C₁₂ hydrocarbyl, or for instance each C₄ - C₁₂ hydrocarbyl, for example a C₁- C₁₂ linear or branched alkyl or C₃ - C₈ cycloalkyl, and may also be joined to form a ring, wherein the optional substituents are for example selected from the group consisting of phenyl and F, Cl, Br, I, -CH₂F, -CH₂Cl, -CH₂Br, -CH₂I, -CN, -NO₂, -CHO, -CO₂H and esters thereof, -CH₂NH₂ and secondary, tertiary and quaternary amines or amides thereof, and -CH₂OH and esters or ethers thereof, and -O-, wherein said ethers, esters and amides for example C₁ - C₆ alkyl ethers, esters and amides.

Hence, the compound with formula (III) is for instance a O-substituted hydroxylamine or N-substituted or N,N-substituted hydrazine. Preferably, the X is N and R₁ and R₂ are each, independently, linear or branched C₁ - C₈, such as C₁ - C₄ alkyl, e.g. methyl, ethyl, n-propyl, isopropyl, cyclopropyl, n-butyl, sec-butyl, isobutyl, or tert-butyl. For example, the compound with formula (III) is 1,1-dimethylhydrazine. Also possible is that R₁ is an optionally substituted benzyl, wherein the optional substituents are for example selected from the group consisting of halogen and C₁ - C₄ alkyl.

N-arylhydrazines and O-aryl-hydroxylamines are less preferred as compound with formula (III) in view of their stability against hydrolysis of the corresponding hydrazones and hydrazines.

In an embodiment, the compound with formula (III) is supported on a heterogeneous support, for example a solid support, such as a polymer support, to allow for easier recovery of the hydrazine. Polymer-supported and resin-supported hydrazine reagents are known as such, for example in the form of beads.

The reaction may be conducted for instance in an appropriate reaction vessel at room temperature, optionally in the presence of a drying agent and optionally in a solvent.

The reaction product has for example a backbone structure having formula (V), with X, R₁ and R₂ as for formula (III):

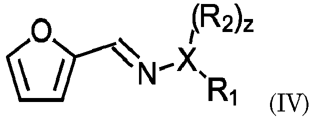

The compound with formula (IV) is a hydrazone or oxime compound and is reacted with a dienophile.

Hence, in a preferred embodiment the compound with formula (IV) has for example the formula: 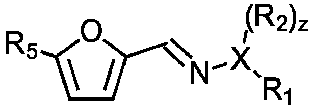 wherein R₅ is an optionally substituted and/or heteroatom containing hydrocarbyl group , as specified for formula (II), and is preferably selected from the group consisting of -CH₂OR₆, -(C=O)R₆, -(C=O)OR₆, wherein R₆ is hydrogen or hydrocarbonyl, preferably C₁ - C₆ hydrocarbonyl.

Advantageously, the reaction of the compound with formula (IV) with a dienophile is not or less liable to retro-reaction, by virtue of the hydrazone or oxime group, thereby allowing the reaction to be driven to higher yield. Optionally, the compound with formula (IV) is obtained and collected as intermediate product, for example by purification, for instance by distillation and crystallization.

The dienophile is for example an alkene or an alkyne. The dienophile suitably has electron withdrawing groups at one or both sides of the acetylene or ethene moiety. The dienophile may for instance have a formula (VI): 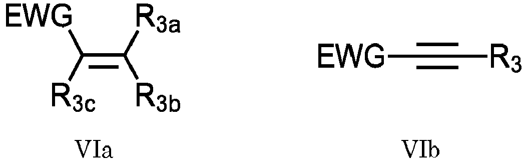 wherein EWG is an electron-withdrawing group and R₃ = H, linear or branched C₁-C₈-alkyl, or EWG, and R₃ₐ, R_{3b} and R_{3c} are for example, independently, EWG, H, C₁-C₈ saturated hydrocarbyl, such as alkyl or C₃- C₈ cycloalkyl, and are optionally substituted, for example only with one or more EWG as substituent; and wherein optionally at least two of EWG, R₃ₐ, R_{3b} and R_{3c} are joined to form a ring. In a preferred embodiment, any and all heteroatoms in the dienophile are provided as part of an EWG.

More preferably EWG = -CN, -NO₂, -CO₂X, -C(O)NX, -C(=NY)X, -CF₃, -CCl₃, -CBr₃, -CI₃, -SO₂X, -SO₃X, -COH, -COX, -COF, -COCl, -COBr, -COI, wherein X and Y are independently H, or C₁-C₈ hydrocarbyl, in particular an linear or branched alkyl or cycloalkyl, optionally substituted with halogens. The dienophile is optionally polymer-supported. However, if the compound with formula (III) is provided on a heterogeneous support, then preferably the dienophile is not supported and is preferably provided as dissolved in the reaction medium.

Alkene dienophiles common in the art may be used, for instance selected from the group consisting of acrylonitrile, maleic anhydride, maleimide, citraconimide, dimethyl fumarate, dimethyl acetylenedicarboxylate, 3-buten-2-one, 1,4-benzoquinone, allyl chloride, maleic acid, itaconic acid, aconitic acid, acrylic acid, crotonic acid, isocrotonic acid, methacrylic acid, tiglic acid, acetylene dicarboxylic methyl ester, vinyl acetate, and esters of maleic and fumaric acids, for example dimethyl maleate and dimethyl fumarate, and alkyl esters of any of such acids. Use of an ester as dienophile, such as with formula (VIa) or (VIb) wherein R₃ is -CO₂X, wherein X is preferably linear or branched C₁-C₈ alkyl, in particular in combination with a Lewis acid catalyst, may be useful to avoid possible interactions between the compound with formula (III) and carboxylic acids.

In some embodiments, the dienophile is an alkene, for example having formula (VI), and the double bond which reacts with the furanic compound with formula (IV) is not part of a ring.

In some embodiments, the dienophile is an alkene with formula (VIa), wherein EWG is -CO₂X, wherein X is H, or linear or branched C₁-C₈ alkyl, preferably wherein X is H, optionally substituted with halogens and optionally polymer-supported, and wherein preferably R₃ₐ, R_{3b} and R_{3c} are selected from hydrogen, methyl and ethyl, or -CO₂X with X as defined for EWG. More preferably, the dienophile is (meth)acrylic acid or alkyl (meth)acrylate. Advantageously, if the dienophile is acrylic acid or acrylate, only a carboxyl group or carboxylate group is introduced into the formed aromatic compound. With furfural as compound with formula (II) and acrylic acid as dienophile, phthalic acid and isophthalic acid may be obtained. With 5-HMF and acrylic acid, for example hemi-mellitic acid may be obtained.

The dienophile may also be a carboxylic acid, wherein EWG is -CO₂X, preferably with a vinyl group. If an carboxylic acid such as maleic acid is used as dienophile, then for example N,N-dimethylhydrazine can be used with for instance furfural and advantageously no drying agent is necessary and/or no solvent is used. If a solvent is used, this may contain water. Suitable solvents include, for example, dichloromethane, toluene, ethyl acetate and 2-sec-butylphenol. Also possible is to run the reaction neat, for instance if the reaction comprises combining furfural with a hydrazine such as N,N-dimethylhydrazine.

In some attractive embodiments, the dienophile is a compound with formula (VIc): 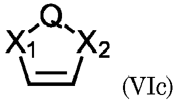 wherein Q is selected from the group consisting of O, N, S, or P, more preferably O; and X₁ and X₂ are independently a -(CH₂)-, -(C=O)-, -(HCOY)- or-(C(OY)₂)-, preferably -(CH₂)- or -(C=O)-; and Y is independently a hydrogen, linear or branched C₁-C₄ hydrocarbyl. Preferably, the dienophile is maleic anhydride. Using furfural and maleic anhydride as dienophile, 3-carboxyphthalic anhydride may be obtained as product. Using 5-HMF and maleic anhydride, 3,6-dicarboxyphthalic anhydride may be obtained as product after hydrolysis and oxidation.

In a preferred embodiment, the dienophile comprises a but-2-ene-1,4-dione moiety included in a 5- or 6-membered ring and is for example maleic anhydride.

Some factors for the choice of the dienophile include the groups to be introduced into the groups that are desirable or acceptable as substituents on the phenyl moiety of the benzene carboxylic product, and the reactivity of the dienophile.

Also possible is the use of ethylene or acetylene, or more generally an unactivated alkene or alkyne. For example an alkene or alkyne not comprising an EWG can be used, for instance an unsubstituted C₂ - C₁₂ alkene or alkyne, that is preferably 1,2-unsaturated.

Although ethylene is normally not very reactive for Diels-Alder reactions, the presence of the hydrazone or oxime group on the furanic compound may enable use of ethylene as dienophile with a variety of catalysts, for instance catalysts similar to those used for the reaction of 2,5-dimethylfuran with ethylene.

In a preferred embodiment, ethylene is used as dienophile. One or more steps of the reaction of the compound with formula (IV) with ethylene, such as the formation of the aromatic compound, may for example be catalysed by a Lewis acid and/or Bronsted acid. For example a supported, or solid or homogenous Bronsted acid may be used. For instance, a porous solid acid material may be used, such as microporous silica, alumina, or aluminasilicate can be used, for example zeolite, or an activated carbon support. Zeolites for example may provide a combination of Lewis acid and Bronsted acid functionality. The reaction is conducted for instance above 200 °C, such as above 250 °C.

If an alkyne dienophile is used, this may for instance be a compound selected from the group consisting of: 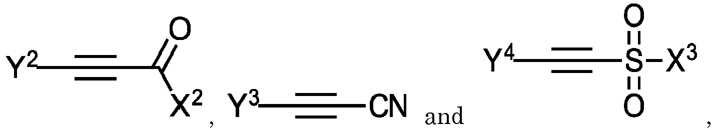 wherein
Y² = H, CH₃ or CO₂X_{A} and
X² = H, CH₃, halogen, OX_{A} or NX_{A}X_{B};,
Y³ = H or CH₃, X³ = CH₃, OX_{A}, NX_{A}X_{B} or halogen,
Y⁴ = H, CH₃, SO₂X³; and X_{A} and X_{B} are independently hydrogen or hydrocarbon, for example C₁ - C₄ alkyl.

If the dienophile is an alkyne, a phenolic compound is obtained. With an acetylene derivative according to formula VIb, wherein R₃ is hydrogen, a phenolic product is obtained, for instance having the formula (VII): 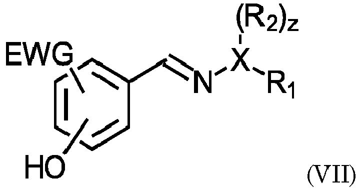 for example with the hydroxyl *para* to the hydrazone or oxime group. In an advantageous embodiment, the dienophile is an alkyl propiolate, more preferably a linear C₁- C₈ alkyl propiolate, in particular methyl propiolate, and such a propiolate group is present as EWG in the compound with formula (VII).

In the present invention, the furanic compound may be considered as a diene. A reaction between a diene and a dienophile is known in the field as a Diels-Alder reaction. As such, for the present invention the reaction of the furanic compound with the dienophile may be referred to as the Diels-Alder reaction. However, it will be appreciated that the present invention is directed to any reaction of the furanic compound with a dienophile, independently of the specific reaction pathway of mechanism involved. For instance, although the Diels-Alder reaction is a concerted reaction, *viz.* a single-step reaction without any intermediate, a non-concerted reaction such as *e.g.* a Friedel-Craft-type pathway is also within the scope of the present invention. Without wishing to be bound by way of theory, an oxabicyclic adduct is formed, having a six-membered ring and a ring with a bridging oxygen atom, in addition to any fused ring from the dienophile. The oxygen-bridging ring may undergo ring-opening, usually *in situ,* for instance if a Lewis acid catalyst is used. The ring opening may also involve a rearrangement. The presence of a hydrazone or oxime group during this ring opening may advantageously increase the rate thereof.

In a particular embodiment of the present invention, the Diels-Alder reaction is catalyzed. Preferably the catalyst is a Bronsted acid or a Lewis acid, or a combination thereof, for example an aprotic Lewis acid, optionally supported on or provided by a solid material or a heterogeneous support, for example silica or a polymer. More preferably the catalyst is a Lewis acid based on a metal, preferably a metal selected from the group consisting of Zn, Al, Sc, B, Fe, Ir, In, Hf, Sn, Ti, Yb, Sm, Cr, Co, Ni, Pb, Cu, Ag, Au, Tl, Hg, Pd, Cd, Pt, Rh, Ru, La, Ce, Pr, Nd, Pm, Eu, Gd, Tb, Dy, Ho, Er, Tm, Lu, V, Mn, Y, Zr, Nb, Mo, Ta, W, Re, Os and combinations thereof. Even more preferably, the catalyst is selected from the group consisting of ZnI₂, ZnBr₂, ZnCl₂, Zn(Ac)₂, Sc(OSO₂CF₃)₃, Y(OSO₂CF₃)₃, AlCl₃, Al(Et)₂Cl, BCl₃, BF₃, B(Ac)₃, FeCl₃, FeBr₃, FeCl₂, Fe(Ac)₂, IrCl₃, HfCl₄, SnCl₄, TiCl₄, clays, zeolites and combinations thereof. Even more preferably, the catalyst is selected from the group consisting of ZnI₂, ZnBr₂, ZnCl₂, Sc(OSO₂CF₃)₃, Y(OSO₂CF₃)₃, AlCl₃, Al(Et)₂Cl, TiCl₄ and combinations thereof. In particular for alkynes as dienophile, yttrium triflate, Y(OSO₂CF₃)₃, is preferred as catalyst. The reaction can also be conducted without catalyst. Without wishing to be bound by way of theory, the catalyst may also catalyze the ring-opening of the oxabicyclic adduct, for instance to a ring-opening dehydration reaction.

If a solid and/or solid supported catalyst is used, such as a zeolite, then the compound with formula (III) is suitable dissolved or at least dispersed in a liquid phase of the reaction medium. In such case, the compound with formula (III) is preferably not volatile and/or contained within the process. For instance, R₁ and R₂ of the compound with formula (III) may comprise each at least 4 or at least 6 carbon atoms, or at least 8 carbon atoms; or R₁ and R₂ in total at least 6 or at least 10 or at least 12 carbon atoms.

In a preferred embodiment, the catalyst is a haloacetic acid, such as a fluoroacetic acid or chloroacetic acid. For instance, the catalyst may be a trihalogenated acetic acid. Good results have been obtained using trifluoroacetic acid as catalyst.

The reaction of the compound with formula (IV) with a dienophile can be performed for example at a temperature ranging from -60 to 350 °C, preferably - 20 to 250 °C, more preferably 20 to 180 °C. The precise temperature depends on the furanic compound and dienophile used. In some embodiments, the reaction is performed at ambient temperature (e.g. between about 5°C and about 35 °C), for example using a Bronsted acid catalyst such as trifluoroacetic acid.

The dienophile is for example provided in about 1 equivalent per furanic compound, for example a molar ratio for example in the range 2:1 to 1:2, more instance 1:1 to 1:1.5 of furanic compound to dienophile. The Diels-Alder reaction may be performed for example at a pressure ranging from 0 - 200 bar, preferably 1 to 50 bar. The Diels-Alder reaction is typically performed in a liquid phase, such as neat, e.g. without solvent, or for example in suitable solvent, preferably in a concentration of 0.1 - 3 M, more preferably about 2 M, of the furanic compound. The solvent is for instance selected from the group consisting of alcohols, esters, ketones, aliphatic hydrocarbons, aromatic hydrocarbons, organic acids, ethers, diprotic apolar solvents, halogenated solvents, nitrated solvents, ionic liquids, organic bases and combinations thereof.

Suitable, the hydrazone or oxime group is subjected to hydrolysis, to provide the corresponding aldehyde and then, in some embodiments, to oxidation to a carboxylic group. For the hydrolytic cleavage of the carbon-nitrogen bond, the liberated nitrogen base is preferably trapped or removed, to drive the reaction forward, for instance by distillation.

The hydrolysis is for example conducted with excess water and for instance catalysed, such as by a Bronsted acid and/or Lewis acid, for instance by a weak Bronsted acid, or an aprotic Lewis acid. The hydrolysis can be conducted using known methods for hydrolysis of compounds comprising a N=C bond, such as hydrazone and oxime compounds, into the corresponding ketones and aldehydes.

With some reagents for the hydrolysis, the oxidation may occur simultaneously. In a preferred embodiment, nitric acid is used for the hydrolysis and/or oxidation. Hence, in a preferred embodiment, the dienophile is maleic anhydride and the compound with a backbone structure according to formula (V) is converted into the product having a backbone structure according to formula (I) in a single step involving hydrolysis and oxidation, using for example nitric acid.

The hydrolysis may for example be conducted using glyoxylic acid and H₂O. For instance, 50% aqueous glyoxylic acid (2 mL) per 1 mmol hydrazone may be used at room temperature. The hydrolysis may also be conducted for example using bismuth trichloride/THF, for example with a small amount of water and under microwave irradiation. Yet a further option may be the use of trimethylsilyl chloride and sodium iodide with MeCN. Optionally, the hydrazone or oxime is subjected to hydrolysis using microwave irradiation and an acid, such as a carboxylic acid, for a period of for example up to 1 hour, up to about 10 minutes or up to about 1 minute.

If the hydrazine or oxime compound with formula III was bonded to a support, this would facilitate the recovery and reuse of the formed hydrazine or hydroxylamine. In a preferred embodiment, the compound with formula (III) is provided on a heterogeneous solid support, and the method comprises recovery of the compound with formula (III) by separation of the solid support material comprising the compound with formula (III) from a liquid phase comprising an aromatic product preferably with formula (I).

The oxidation of the aldehyde into the corresponding carboxylic acid may be conducted under relatively mild conditions and is known as such in the art. For example, Ag₂O or, potassium peroxymonosulfate could be used.

Also possible is oxidation of the aromatic hydrazine to the corresponding nitrile, followed by hydrolysis of the nitrile.

The method of the invention advantageously allows for preparing benzene di-carboxylic acids, such as phthalic acid, isophthalic acid and terephthalic acid. Also hemi-mellitic may be obtained. Generally, for example benzene monocarboxylic acids, such as benzoic acid, benzene dicarboxylic acids, benzene tricarboxylic acids, and benzene tetracarboxylic acids may be obtained, as well as for example hydroxybenzene mono di, tri, and tetra carboxylic acid, as well as esters and anhydrides thereof. In some embodiments, the benzene carboxylic acid has no other substituents than the carboxylic group and optionally the hydroxyl groups.

The method or at least the reaction with the dienophile may be performed batch-wise or preferably in a continuous process. For a batch process, the reaction is optionally performed as a one pot synthesis, for instance the reaction with the compound with formula (III) and the reaction with the dienophile, by sequential addition of reagents, for example without work-up. This is optionally also possible if the feed containing the compound with backbone structure with formula (II) also comprises water. The feed is for example an effluent from a reactor, for instance from a biphasic reactor, that is optionally filtrated or phase separated to provide an organic phase used as feed for the method of the invention.

Generally, the feed for the method, or for the reaction with the compound with formula (III), that comprises the furanic compound, is for example an effluent from a reactor for the catalytic dehydration of biomass. The feed may comprise an organic solvent and/or water, such as for example obtained from a biphasic reactor. The feed may in particular comprise a mixture of furanic compounds.

Optionally, a solid-supported compound with formula (III) is used, wherein the solid material may for example be immobilized. The solid-material comprising the compound with formula (III) may be supplied as particles, powder, or in granular form and is for example a polymer-supported hydrazine or hydroxylamine. The solid supported compound is for example used as a mobile phase and for example passed through a zone for reaction with a dienophile and subsequently through one or more zones for hydrolysis and oxidation, and then recovered and reused.

Preferably, the dienophile comprises a vinyl group, and preferably the dienophile is a carboxylic acid. More preferably, the dienophile is acrylic acid or an ester thereof, as it allows for introducing carboxylic groups into the formed aromatic compound.

The dienophile may also be an acrylate ester, preferably an alkyl acrylate ester, for instance with a C₁ - C₆ alkyl group, more in particular methyl acrylate.

If furfural is used and acrylic acid and esters thereof as dienophile, the Diels-Alder reaction may give the following compounds: 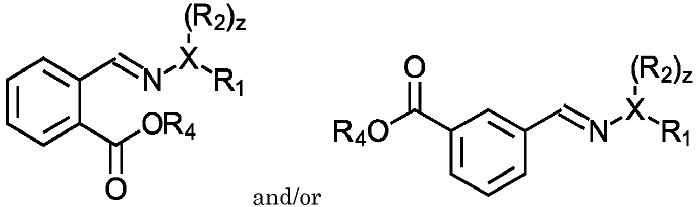 wherein X, R₁, R₂ and z are as defined before, and R₄ is hydrogen or a hydrocarbyl, preferably an alkyl or cycloalkyl with 1 to 6 carbon atoms, and wherein preferably X = N and preferably R₁ = R₂ = methyl.

These compounds can be converted into the corresponding dicarboxylic acid using hydrolysis and oxidation of the hydrazone or oxime group, for instance as described above.

In yet a further embodiment, the hydrazone or oxime furanic compound has the formula (VIII), for instance obtained by reacting 5-HMF with the compound with formula (III): 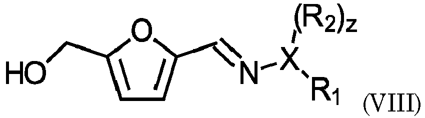 wherein X, R₁ and R₂, and z are as defined before, and preferably X = N and preferably R₁ = R₂ = methyl, and is reacted with acrylic acid and esters to give a compound with the formula: 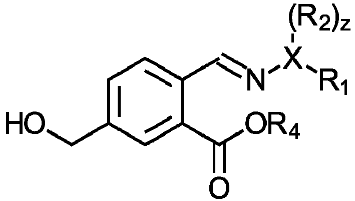 wherein R₄ is as defined before, and wherein the method generally further comprises oxidation of the hydroxyl to carboxylic acid.

In yet a further embodiment, the compound with formula (III) is an oxime wherein X = O, such that a hydroxylamine is used giving an oxime compound with a backbone structure having the formula (IX):

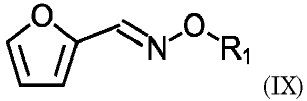

Particularly preferred as compound with formula (III) is *O*-benzyl hydroxylamine, that may for instance be reacted with furfural, for example using pyridine in ethanol at room temperature. Such reaction is described in J. Org. Chem. 73(4) 1264-1269 (2008).

For an oxime compound according to formula (IX), the dienophile may for instance be an alkyne, more preferably an alkyl propiolate, such as a C₁ - C₄ alkyl propiolate, in particular methyl propiolate.

In an embodiment, methyl propiolate is reacted with furfural-benzyloxime, for instance in an organic polar solvent such as ethyl acetate, using for example a Lewis acid metal as catalyst, such as aluminium chloride. Also possible is that 2,5-furandicarboxaldehyde furfural, 5-hydroxymethylfurfural, methoxymethylfurfural, and/or chloromethylfurfural is reacted with a hydroxylamine with formula (III) and a propiolate is used as dienophile, more preferably an alkyl propiolate such as methyl propiolate.

In a preferred embodiment, the product is terephthalic acid and the dienophile is ethylene. More preferably, the compound with formula (II) is 5-hydroxymethylfurfural, methoxymethylfurfural or 2,5-furandicarboxaldehyde. Preferably, the reaction of the dienophile with the compound with a backbone structure according to formula (IV) is catalysed by a Lewis and/or Bronsted acid.

In a preferred embodiment, the invention relates to a terephthalic acid production process comprising reacting a hydrazone or oxime substituted furan with ethylene under cycloaddition reaction conditions and in the presence of a catalyst to produce a bicyclic ether, dehydrating the bicyclic ether to produce a hydrazone or oxime substituted phenyl and converting the substituted phenyl to terephthalic acid. Examples of cycloaddition reaction conditions are e.g. a temperature of about 100 °C to about 300 °C, an ethylene partial pressure from about 1 to about 100 bar, and/or a reactor residence time of for example less than 1 hour. The process may be performed batch-wise or in a continuous process.

Possible catalysts include activated carbon, silica, alumina, a zeolite, or a molecular sieve. For example, the catalyst may be a heterogeneous material providing Lewis and/or Bronsted acid functionality. Optionally, at least 6 of the carbon atoms of the obtained terephthalic acid are derived from one or more renewable feed stocks. Optionally, the furan is not methyl substituted when reacted with ethylene. Preferably, the furan is substituted at the 2 position with a hydrazone or oxime functionality when reacted with ethylene.

Yet a further embodiment relates to a process of preparing a hydrazone and/or oxime substituted phenylic compound, comprising reacting a hydrazone and/or oxime substituted furanic compound, preferably substituted with hydrazone or oxime at the 2 position, and preferably obtained from renewable feedstock, with a non-substituted and/or unactivated alkyne or alkene compound, in particular acetylene or ethylene, under cycloaddition reaction conditions, followed by or further comprising dehydration of the adduct to give a phenylic compound. The phenylic compound may be reacted further for instance to a carboxylic acid or to 1,4 cyclohexane dimethanol. Preferred features of such process are as for the preparation of benzene carboxylic acids.

Yet a further preferred method comprises reacting 5-HMF to 2,5-furandicarboxaldehyde (DFF), reacting DFF with a hydrazine or hydroxylamine compound according to formula (III), wherein X is preferably N and preferably R₂ and R₁ are both methyl, and the compound according to formula (III) with ethylene as dienophile to give a hydrazone or oxime benzaldehyde compound, and usually reacting the benzaldehyde compound to terephthalic acid, such as by hydrolysis and oxidation. The method may also start with reacting DFF with hydrazine or the oxime compound.

In yet a further embodiment, 5-HMF is directly reacted with a hydrazine or oxime compound according to formula (III), wherein X is preferably N and preferably R₂ and R₁ are both methyl, to give a hydrazone or oxime benzyl alcohol compound, that can be converted to terephthalic acid by relatively mild hydrolysis and oxidation. If instead of ethylene, acetylene is used, 2-hydroxyphtalic acid may be obtained.

The invention also relates to the use of hydrazone or oxime substituted furan compounds for the preparation of phenylic compounds by the reaction with ethylene or acetylene. This reaction gives a hydrazone or oxime substituted phenylic compound, that may for instance be useful as precursor or intermediate for the preparation of compounds such as monomers for various polymers, in particular polyesters, such as polyesters having monomers or co-monomers comprising an aromatic ring, such as polyethylene-terephthalate.

The invention also relates to the use of hydrazine or hydroxylamine compounds for the activation of biomass derived compounds for Diels Alder reactions, in particular for reaction of biomass derived dienes with ethylene or acetylene. Optionally, this use involves solid-supported hydrazone or hydroxylamine compounds, and a heterogeneous solid material comprising hydrazine and/or hydroxylamine groups is contacted with biomass derived compounds, in particular furfural carbaldehyde compounds.

In an aspect, the invention also relates to use of hydrazine and/or hydroxylamine compounds that are preferably solid-supported, for the activation of furanic compounds in an effluent of the preferably catalytic dehydration of carbohydrates, preferably without intermediate reduction of the furanic compounds, for reaction of the furanic compound with a dienophile to give aromatic compounds. Preferably the effluent is a liquid stream obtained from a reactor for the dehydration of carbohydrate. Preferably, the carbohydrate dehydration and the reaction with the hydrazine and/or hydroxylamine are carried out simultaneously in separate zones of a continuous process.

In some embodiments, the method is for the preparation of an ester having a backbone structure according to formula (Ia): 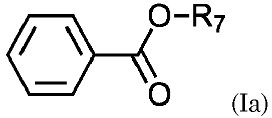 wherein R₇ is an optionally substituted and/or heteroatom containing hydrocarbyl group. R₇ can for example be selected from the group of C₁ - C₂₀ linear or branched alkyl, alkenyl, alkynyl, cycloalkyl, aryl, aralkyl and alkaryl, optionally containing heteroatoms and/or substituents for example selected from the group consisting of F, Cl, Br, I, -CH₂F, -CH₂Cl, -CH₂Br, -CH₂I, -CN, -NO₂, -CHO, -CO₂H and esters thereof, -CH₂NH₂ and secondary, tertiary and quaternary amines or amides thereof, and -CH₂OH and esters or ethers thereof; wherein said heteroatoms are optionally selected from O, N, S, and P. Generally, the corresponding amides can also be obtained as product.

The method suitably comprises converting the compound with a backbone structure according to formula (V) into an ester, preferably by hydrolysis and oxidation. Preferably, the method comprises *in situ* hydrolysis and esterification of the compound with a backbone structure according to formula (V).

More generally, the hydrolysis and oxidation may involve the formation of a hemiacetal and oxidation of the hemiacetal, optionally followed by hydrolysis of the ester to the corresponding carboxylic acid, for instance through a saponification procedure.

Some illustrative non-limiting reaction schemes of aspects of the invention are shown below.

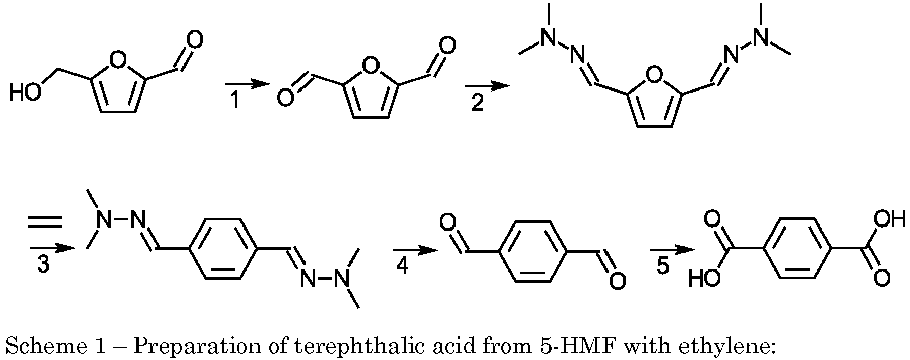

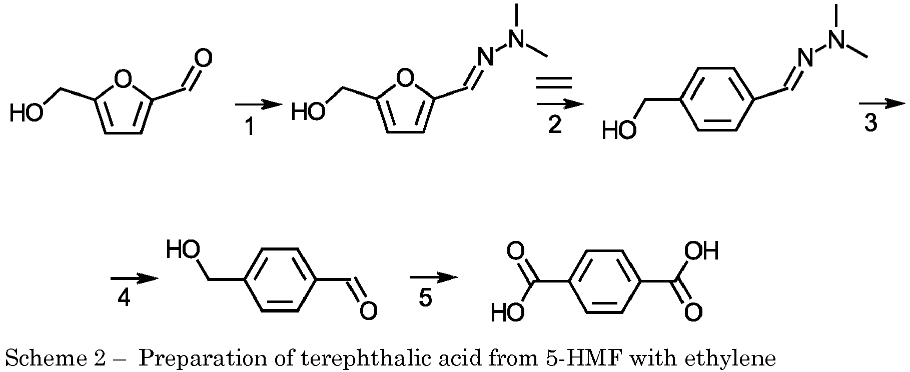

Optionally, a combination of hydrazone and oxime can be used. Optionally, a combination of an alkene and alkyne dienophile may be used. Any references to mechanisms and intermediate are included without wishing to be bound by way of theory.

As used herein, with "backbone structure" is meant that the formula schematically represent a core structure of a group of compounds, which are optionally additionally substituted with any group or atom at any position. Moreover, the backbone structures include at least any diastereomers, such as, for example, *cis* / *trans* isomers. Any references to compounds with formula (I), (II), (IV) and (V) are to backbone structures optionally having substituents, even if not expressly recited. Any reference to hydrocarbyl substituents includes heteroatom containing hydrocarbyl substituents, such as hydrocarbyl groups containing for example one or more atoms selected from the group of O, N, S and P, for example a substituent selected from the group consisting of alkyl, aryl, alkenyl, alkynyl, cycloalkyl, aralkyl and alkaryl, optionally containing heteroatoms.

### Examples

The invention will now be further illustrated by the following non-limiting examples of various steps of the methods of the invention.

### Example 1

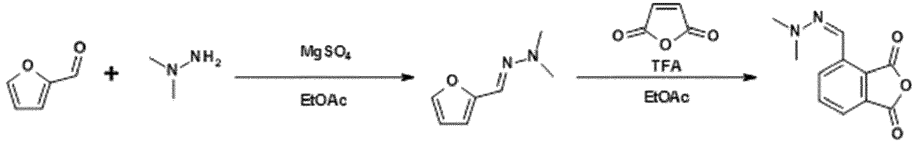

To a reactor was added magnesium sulfate (14.44g, 1eq.) and ethyl acetate (58.6mL), and the mixture was stirred. To this was added furfural (11.53g, 9.94mL, 1eq.), and after 2 minutes 1,1-dimethylhydrazine (7.284g, 9.22mL, 1.01eq) was then added dropwise, and the reaction was stirred at room temperature for 16 hours. The reaction mixture was filtered to remove the solids, then the cake was washed with ethyl acetate (2x20mL). To the resulting yellow solution of furfural-dimethylhydrazone was added maleic anhydride (15.00g) and ethyl acetate (30mL), and the mixture was stirred vigorously. After 3 minutes, trifluoroacetic acid (684mg, 459µL) was added, and the reaction was heated to 60°C and held for 3 hours. The reaction mixture was cooled to 0°C and stirred for 20 minutes, then the solid was isolated by vacuum filtration. The filtrate appeared to contain product. The cake was washed with ice-cold EtOAc (2x30ml), then dried in a vacuum oven to yield a bright yellow solid (18.5g, yield 71% over 2-steps).

In a variation, the reaction could be stirred for about 3 hours. Magnesium sulfate is suitable not required for a good yield of hydrazone and is optionally omitted. A wide variety of solvents were found to be suitable for the formation of the hydrazone. The Diels-Alder reaction can be performed in a range of solvents. The Diels-Alder reaction proceeds without TFA, but slowly. The Diels-Alder was also observed to proceed at ambient temperature. Further results indicate that the reaction can be telescoped even when the solvent is wet from the first reaction. For example a one-pot synthesis with reagents added to a reactor sequentially and without work-up can be used. The reaction mixture was darker in color than with dry solvent for the DA reaction, but the reaction is quite clean by HPLC.

### Example 2

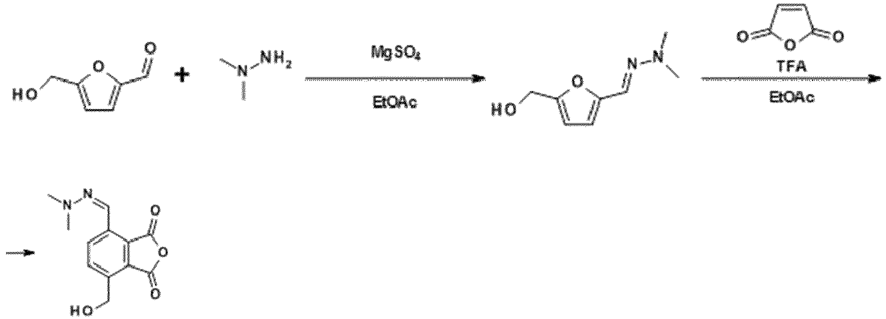

To a reactor was added magnesium sulfate (7.37g, 1eq.) and ethyl acetate (29.9mL), and the mixture was stirred. To this was added HMF (7.718g, 1eq.), and after 2 minutes 1,1-dimethylhydrazine (3.715g, 4.702mL, 1.01eq) was then added dropwise, and the reaction was stirred at room temperature for 16 hours (3 hours is expected to be sufficient). The reaction mixture was filtered to remove the solids, then the cake was washed with ethyl acetate (2x20mL). To the resulting orange solution of HMF-dimethylhydrazone was added maleic anhydride (7.652g, 1.275eq.) and ethyl acetate (30mL), and the mixture was stirred vigorously. After 3 minutes, trifluoroacetic acid (349mg, 234µL, 0.05eq.) was added, and the reaction was heated to 60°C and held for 1 hour. The reaction mixture was cooled to ambient temperature then washed with saturated sodium bicarbonate solution (30mL), then water. The resulting organic solution was dried over sodium sulfate, filtered and reduced to yield and oil. This was purified over silica, eluting with ethyl acetate/heptane. The appropriate fractions were collected and reduced to yield the desired product as an orange oil (1.975g, 13% over 2-steps).

### Example 3

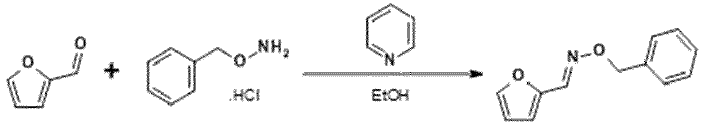

To a reactor was added O-benzylhydroxylamine hydrochloride (766mg, 1.2eq) and absolute ethanol (3ml), and the mixture was stirred vigorously, then pyridine (1.264g, 1.293mL, 4eq.) was added dropwise. To this was added furfural (384mg, 331µL, 1eq.) dropwise. This was stirred at room temperature for 4 hours (the reaction is expected to be complete in minutes, e.g. less than 15 minutes), then the ethanol was removed by rotary evaporation to yield a white solid and light yellow liquid. To this dissolved/suspended in dichloromethane (15ml), and the mixture extracted twice with a 5% citric acid solution (30ml). The combined aqueous was back extracted with dichloromethane (15ml) and the combined organics dried over sodium sulfate, filtered, and reduced to yield a light yellow oil. This was then purified on over silica eluting with an n-Hexane:EtOAc solvent system. Appropriate fractions were collected and reduced to yield the desired product as a clear oil (719mg, 103%).

### Example 4

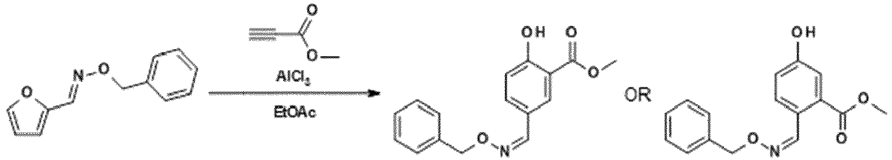

To a reactor was charged methyl propiolate (26.6mg, 28.2µL, 1.275eq.) and ethyl acetate (100µL), and stirring was started. To this was added a solution of the furfural-benzyloxime (50mg, 1eq.) in ethyl acetate (100µL) dropwise over 5 mins. The reaction mixture was cooled to 0°C and then aluminium chloride (40mg) was added. The cooling was removed and the reaction allowed to warm to room temperature and stirred for 1 hour. The reaction mixture was cooled to 0°C then quenched by the dropwise addition of water (2ml). Ethyl acetate (2ml) was added and the organics were separated. The aqueous was extracted with a second portion of ethyl acetate (5ml). The combined organics were washed with water, dried over sodium sulfate, filtered and reduced to an oil by rotary evaporation. Purified over silica eluting with an n-Hexane:EtOAc solvent system. Appropriate fractions were collected and reduced to yield the desired product as brown oil (5mg, 7%).

### Example 5

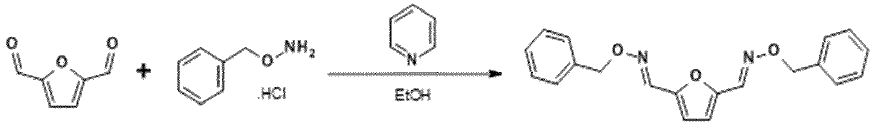

To a reactor was added O-benzylhydroxylamine hydrochloride (766mg, 1.2eq) and absolute ethanol (3ml), and the mixture was stirred vigorously, then pyridine (1.264g, 1.293mL, 4eq.) was added dropwise. To this was added 2,5-furandicarboxaldehyde (248mg, 1eq.) dropwise. This was stirred at room temperature for 4h (the reaction is expected to be complete in minutes, e.g. less than 15 minutes). Then the ethanol was removed by rotary evaporation to yield a white solid and light yellow liquid. To this dissolved/suspended in dichloromethane (15ml), and the mixture extracted twice with a 5% citric acid solution (30ml). The combined aqueous was back extracted with dichloromethane (15ml) and the combined organics dried over sodium sulfate, filtered, and reduced to yield a light yellow oil. This was then purified over silica eluting with an n-Hexane:EtOAc solvent system. Appropriate fractions were collected and reduced to yield the desired product as a light yellow oil (646mg, 97%).

### Example 6

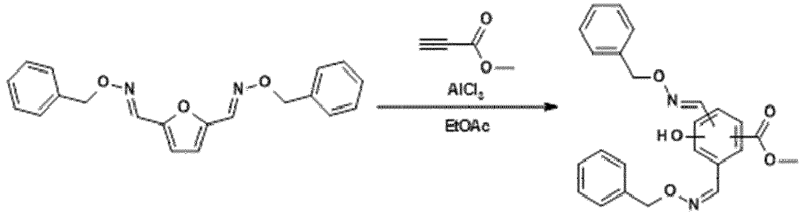

To a reactor was charged methyl propiolate (32.0mg, 33.9µL, 1.275eq.) and ethyl acetate (250µL), and stirring was started. To this was added a solution of the 2,5-furandicarboxaldehyde-bis-benzyloxime (100mg, 1eq.) in ethyl acetate (250µL) dropwise over 5 mins. The reaction mixture was cooled to 0°C, then aluminium chloride (100mg, 2eq.) was added. The cooling was removed and the reaction allowed to warm to room temperature and stir for 1 hour. The reaction mixture was cooled to 0°C then quenched by the dropwise addition of water (2ml). Ethyl acetate (2ml) was added and the organics were separated. The aqueous was extracted with a second portion of ethyl acetate (5ml). The combined organics were washed with water, dried over sodium sulfate, filtered and reduced to an oil by rotary evaporation. Purified over silica eluting with an n-Hexane:EtOAc solvent system. Appropriate fractions were collected and reduced to yield 3 separate region-isomers of the desired product:
Product 1 isolated as a yellow oil (25mg, 20%).
Product 2 isolated as a yellow oil (20mg, 16%).
Product 3 isolated as a brown oil (15mg, 12%).
Overall yield = 60mg, 48%.

### Example 7

To a reactor was charged 3-formylphthalic anhydride-dimethylhydrazone (2.5g, 1eq.) and 10% aqueous nitric acid (25mL), and this was heated to 100°C with vigorous stirring. After ∼30 minutes, when all of the solid had dissolved and no more gas was given off, the reactor was configured to distill water. The reaction mixture was reduced to ∼10mL in volume, then cooled to around 75°C. The surface of the reactor was scratched with a pipette to induce crystallization. The solid which formed was isolated by filtration. The cake was washed with ice-cold water (2x10ml), then dried in a vacuum oven to yield a cream colored solid (1.97g; 89%). The product is possibly hemi-mellitic anhydride.

## Claims

1. A method of preparing a compound having a backbone structure according to formula (I), 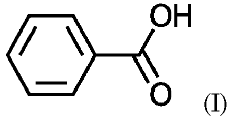 or esters or anhydrides thereof from biomass-derived compounds, comprising reacting a biomass-derived compound having a backbone structure according to formula (II): 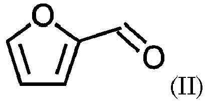 with a compound with formula (III): 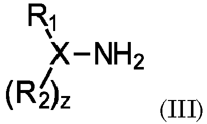
wherein X is O and z is 0, or X is N and z is 1,
wherein R₁ and R₂ are each independently an optionally substituted and/or heteroatom containing hydrocarbyl group or a link to a heterogeneous support, and
R₂ can also be hydrogen,
to give a compound with a backbone structure according to formula (IV): 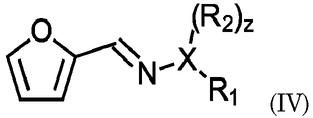
and reacting the compound with a backbone structure according to formula (IV) with a dienophile to give a compound with a backbone structure according to formula (V): 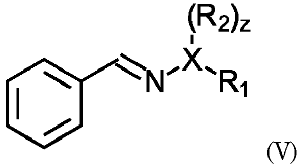 and converting said compound into the compound of formula (I) by hydrolysis and oxidation.

2. A method according to claim 1, wherein said compound having a backbone structure according to formula (II) is furfural, furan-2,5-dicarbaldehyde, methoxymethylfurfural, chloromethylfurfural or 5-hydroxymethylfurfural, or a mixture thereof.

3. A method according to claim 1 or 2, wherein the compound having a backbone structure according to formula (I) is a benzene dicarboxylic acid, a benzene tricarboxylic acid, or ester or anhydride equivalents thereof, or a benzene tetracarboxylic acid, or, when the dienophile is ethylene, benzoic acid or esters thereof.

4. A method according to any of claims 1-3, wherein the reaction of the dienophile with the compound with a backbone structure according to formula (IV) is catalysed by a Lewis and/or Bronsted acid.

5. A method according to any of claims 1-4, wherein the dienophile is ethylene.

6. A method according to claim 5, wherein the compound with formula (II) is 5-hydroxymethylfurfural, methoxymethylfurfural, chloromethylfurfural or 2,5-furandicarboxaldehyde, and the product having a backbone structure according to formula (I) is terephthalic acid.

7. A method according to claim 6, wherein a Bronsted acid and/or a Lewis acid supported on a heterogeneous support or provided by a solid material is used as catalyst for the reaction with the dienophile and/or the formation a phenyl ring.

8. A method according to any of claims 1-3, wherein the dienophile is an alkyne.

9. A method according to claim 8, wherein the compound with formula (III) is a hydroxylamine wherein X is O.

10. A method according to claim 9, wherein 2,5-furandicarboxaldehyde, furfural, 5-hydroxymethylfurfural, methoxymethylfurfural, and/or chloromethylfurfural is reacted with a hydroxylamine with formula (III) and the dienophile is a propiolate.

11. A method according to any of claims 1-3, wherein the dienophile is acrylic acid or an acrylate ester.

12. A method according to any of claims 1-11, for the preparation of an ester having a backbone structure according to formula (Ia): 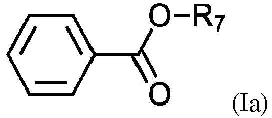 wherein R₇ is an optionally substituted and/or heteroatom-containing hydrocarbyl group, the method comprising converting the compound with a backbone structure according to formula (V) into an ester by hydrolysis and oxidation.

13. A method according to any of claims 1-12, wherein the compound with formula (III) is provided on a heterogeneous solid support, and the method comprises recovery of the compound with formula (III) by separation of the solid material comprising the compound with formula (III) from a liquid phase comprising an aromatic product with formula (I).

14. A method according to any of claims 1-13, wherein the compound having a backbone structure according to formula (II) is provided as an effluent from a reactor for the catalytic dehydration of carbohydrates, wherein the effluent comprises water.

15. A method according to any of claims 1-4, wherein the dienophile is maleic anhydride and wherein the compound with a backbone structure according to formula (V) is converted into the product having a backbone structure according to formula (I) in a single step involving hydrolysis and oxidation.

16. Use of a solid-supported hydrazine and/or hydroxylamine compound for the activation of furanic compounds in an effluent of the catalytic dehydration of carbohydrates, without intermediate reduction of the furanic compound, for reaction with a dienophile to give aromatic compounds.
